(19) Europäisches Patentamt
European Patent Office
Office européen des brevets

(11) Veröffentlichungsnummer : **0 358 108 B1**

(12) # EUROPÄISCHE PATENTSCHRIFT

(45) Veröffentlichungstag der Patentschrift : **15.04.92 Patentblatt 92/16**

(51) Int. Cl.$^5$ : **C07D 209/48,** A01N 43/38

(21) Anmeldenummer : **89116076.4**

(22) Anmeldetag : **31.08.89**

(54) **5-(N-3,4,5,6-Tetrahydrophthalimido)-zimtsäurederivate.**

Die Akte enthält technische Angaben, die nach dem Eingang der Anmeldung eingereicht wurden und die nicht in dieser Patentschrift enthalten sind.

(30) Priorität : **09.09.88 DE 3830733**

(43) Veröffentlichungstag der Anmeldung : **14.03.90 Patentblatt 90/11**

(45) Bekanntmachung des Hinweises auf die Patenterteilung : **15.04.92 Patentblatt 92/16**

(84) Benannte Vertragsstaaten : **BE CH DE ES FR GB IT LI NL**

(56) Entgegenhaltungen :
**EP-A- 0 049 508**
**EP-A- 0 240 659**
**DE-A- 3 900 353**
**GB-A- 2 071 100**
**CHEMICAL ABSTRACTS, Band 105, 1986, Seite 606, Zusammenfassung Nr. 60524v, Columbus, Ohio, US; & JP-A-61 27 962 (MITSUBISHI CHEMICAL INDUSTRIES CO., LTD) 07-02-1986**

(73) Patentinhaber : **BASF Aktiengesellschaft**
**Carl-Bosch-Strasse 38**
**W-6700 Ludwigshafen (DE)**

(72) Erfinder : **Plath, Peter, Dr.**
**Hans-Balcke-Strasse 13**
**W-6710 Frankenthal (DE)**
Erfinder : **Eicken, Karl, Dr.**
**Am Huettenwingert 12**
**W-6706 Wachenheim (DE)**
Erfinder : **Rueb, Lothar, Dr.**
**Am Schoeneck 11**
**W-6720 Speyer (DE)**
Erfinder : **Schwalge, Barbara, Dr.**
**Adolf-Diesterweg-Strasse 77**
**W-6700 Ludwigshafen (DE)**
Erfinder : **Westphalen, Karl-Otto, Dr.**
**Mausbergweg 58**
**W-6720 Speyer (DE)**
Erfinder : **Wuerzer, Bruno, Dr.**
**Ruedigerstrasse 13**
**W-6701 Otterstadt (DE)**

EP 0 358 108 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft 5-(N-3,4,5,6-Tetrahydrophthalimido)-zimtsäurederivate der Formeln Ia und Ib

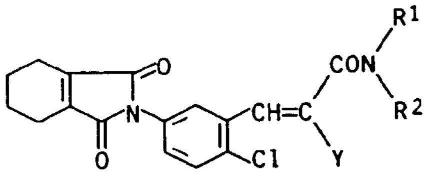

Ia

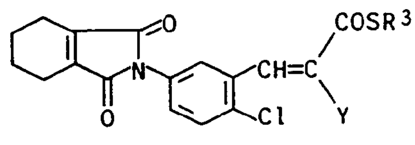

Ib

in denen die Substituenten folgende Bedeutung haben:

Y Chlor, Brom oder eine $C_1$-$C_4$-Alkylgruppe

$R^1$ Wasserstoff,

eine $C_1$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_3$-$C_6$-Alkenylgruppe,

eine $C_3$-$C_6$-Alkinylgruppe,

ein Phenylring oder ein Benzylrest, wobei die Ringe ein bis drei der folgenden Reste tragen können: Halogen, $C_1$-$C_4$-Alkyl, $C_1$-$C_4$-Alkoxy, $C_1$-$C_4$-Halogenalkyl, Cyano, Nitro, $C_1$-$C_4$-Halogenalkoxy, N-$C_1$-$C_4$-Alkylamino, N,N-Di-$C_1$-$C_4$-Alkylamino und/oder $C_1$-$C_4$-Alkylthio,

$R^2$ eine $C_5$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_1$-$C_6$-Alkoxygruppe,

eine $C_3$-$C_6$-Alkenylgruppe,

eine $C_3$-$C_6$-Alkenyloxygruppe,

eine $C_3$-$C_6$-Alkinylgruppe,

eine $C_3$-$C_6$-Alkinyloxygruppe,

eine Phenyl-, Phenoxy-, Benzyl- oder Benzyloxyrest, wobei diese Reste ein bis drei der folgenden Substituenten tragen können: Halogen, $C_1$-$C_4$-Alkyl, $C_1$-$C_4$-Alkoxy, $C_1$-$C_1$-Alkylthio, $C_1$-$C_4$-Halogenalkyl, Cyano, Nitro, $C_1$-$C_4$-Halogenalkoxy, N-$C_1$-$C_4$-Alkylamino und/oder N,N-Di-$C_1$-$C_4$-alkylamino,

$R^3$ eine $C_1$-$C_6$-Alkylgruppe,

eine $C_2$-$C_6$-Alkylgruppe, welche ein bis drei der folgenden Substituenten tragen kann: $C_1$-$C_4$-Alkoxy, $C_1$-$C_4$-Alkylthio und/oder Hydroxy,

wobei $R^1$ nicht Wasserstoff bedeutet, wenn Y Chlor und Brom bedeutet.

Weiterhin betrifft die Erfindung Verfahren zur Herstellung dieser Verbindungen sowie deren Verwendung als Herbizide.

Aus der japanischen Offenlegungsschrift JP-OS Kokai 27962/1986 sind Ester und Amide der Struktur I' bekannt,

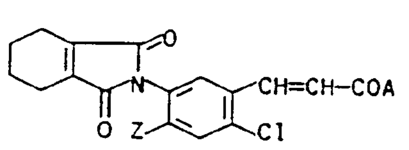

I'

in der A beispielsweise $-OCH_3$, $-NHCH_3$, $-N(CH_3)_2$ oder $-N(CH_2CH=CH_2)_2$ und Z Wasserstoff oder Fluor bedeutet.

Ferner sind in der EP-A-240 659 Verbindungen der Formel I" beschrieben,

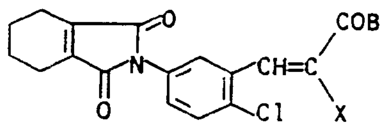

I''

in der B unter anderem $NR^4R^5$ mit $R^4$ und $R^5$ z.B. unabhängig voneinander Wasserstoff und $C_1$-$C_4$-Alkylgruppe und X beispielsweise Wasserstoff, $-CH_3$, -Cl und -Br bedeutet.

Schließlich beschreibt die GB-A-2 071 100 Verbindungen der Formel I'''

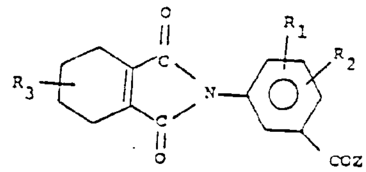

I'''

in der Z unter anderem eine Alkoxy- oder eine Alkylthiogruppe bedeutet.

Besonders für die Anwendung neben Kulturpflanzen sind jedoch Verbindungen wünschenswert, die bei geringerer Aufwandmenge höhere Selektivität gegenüber Kulturpflanzen aufweisen.

Der Erfindung lag daher die Aufgabe zugrunde, entsprechende Substanzen zu finden und zu synthetisieren.

Es wurde gefunden, daß die eingangs definierten 5-(N-3,4,5,6-Tetrahyrophthalimido)-zimtsäurederivate der Formeln Ia und Ib besonders im Nachauflaufverfahren eine vorteilhafte herbizide Wirkung haben und gegen eine Reihe von Kulturpflanzen selektiv sind.

Außerdem wurden Verfahren zur Herstellung der Verbindungen Ia und Ib gefunden.

Man erhält die Verbindungen Ia beispielsweise, indem man 3,4,5,6-Tetrahydrophthalsäureanhydrid mit der äquivalenten Menge eines 5-Aminozimtsäureamids der Formel V in einem inerten organischen Lösungsmittel umsetzt.

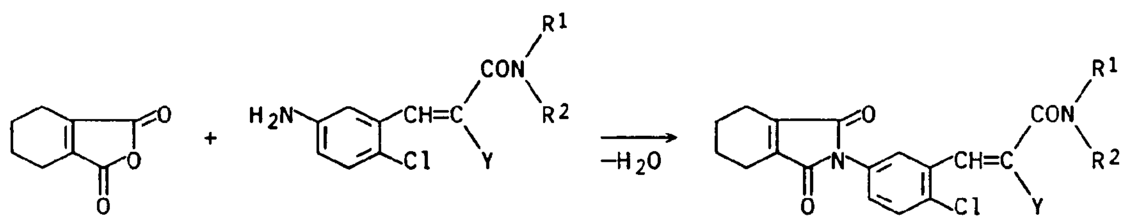


V Ia

Als Lösungsmittel dienen z.B. niedere Alkansäuren wie Essigsäure, Propionsäure und Isobuttersäure, sowie deren Ester wie Essigsäureethylester, höhersiedenden Kohlenwasserstoffe wie Toluol und Xylol und/oder Dimethylformamid. Die Umsetzung erfolgt in der Regel bei Temperaturen zwischen 25°C und dem Siedepunkt der jeweiligen Reaktionsmischung, vorzugsweise zwischen 70 und 140°C. Beim Arbeiten in einem aprotischen Lösungsmittel empfiehlt es sich, das Wasser fortlaufend zu entfernen.

Die Anilinderivate der Formel V können in an sich bekannter Weise aus den entsprechenden Nitrophenylderivaten IV entweder durch Reduktion mit anorganischen Verbindungen wie Zinn-II-salzen oder Eisen oder, sofern Y nicht Brom bzw. Chlor bedeutet, durch katalytische Hydrierung an Metallkontakten wie Raney-Nickel, Palladium und Platin erhalten werden.

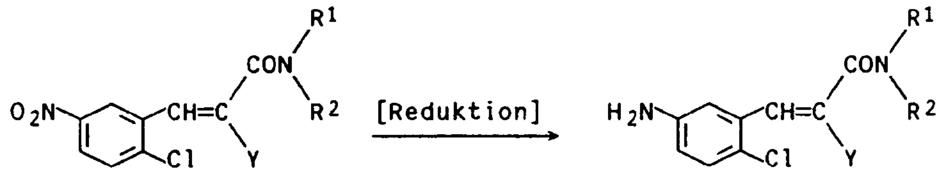


IV V

Die benötigten Nitrophenylderivate sind auf verschiedene Weise herstellbar, z.B.:

a) sofern Y $C_1$-$C_4$-Alkylgruppe bedeutet, indem man ein entsprechendes Nitrozimtsäurechlorid II (EPA 240 659) in einem inerten organischen Lösungsmittel, welches Wasser enthalten kann, in Anwesenheit einer Base mit einem Amin III umsetzt.

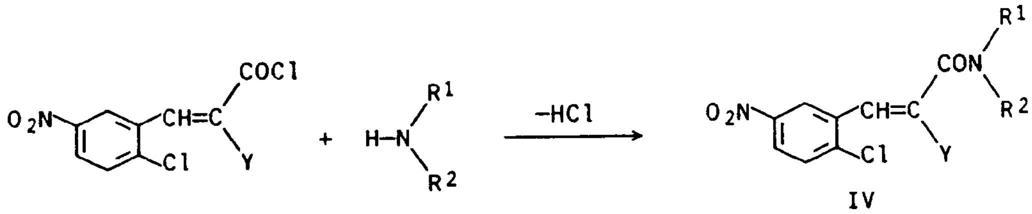


IV

II III

Die Umsetzungstemperatur liegt im allgemeinen zwischen -15 und 50°C, vorzugsweise -10 bis 40°C. Geeignete Lösungsmittel sind beispielsweise Tetrahydrofuran, Dioxan, 1,2-Dimethoxyethan und Acetonitril. Als Basen eignen sich u.a. das Amin selbst im Überschuß oder tertiäre Amine wie Triethylamin, Diisopropylethylamin, N,N-Dimethyl-p-aminopyridin, N-Methylpyrrolidin, N,N,N'N'-Tetramethylethylendiamin, 1,5-Diazabicyclo(4.3.0)non-5-en und 1,8-Diazabicyclo(5.4.0)undec-7-en.

Vorzugsweise arbeitet man in Tetrahydrofuran oder Dioxan, wobei das Amin als wäßrige Lösung oder als Lösung in einem der obengenannten Ether zugegeben werden kann und mit Triethylamin als Base.

b) sofern Y Chlor oder Brom (Hal) bedeutet, indem man ein Nitrozimtsäureamid der Formel IVa in einem inerten organischen Lösungsmittel mit dem entsprechenden Halogen umsetzt.

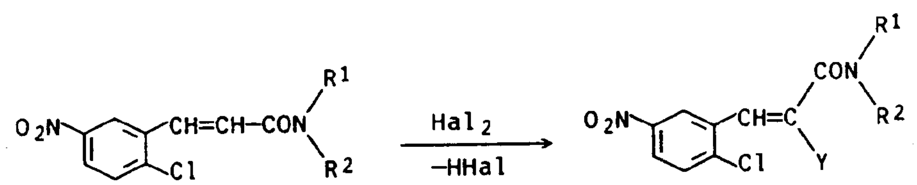


IVa IV

Die Reaktion wird in der Regel bei Temperaturen von 0 bis 60°C, vorzugsweise 15 bis 40°C durchgeführt. Geeignete Lösungsmittel sind beispielsweise Methylenchlorid, Chloroform, Tetrachlorkohlenstoff, 1,1,1-Trichlorethan und Chlorbenzol, wobei Methylenchlorid, Chloroform und 1,1,1-Trichlorethan bevorzugt werden.

Die Verbindungen der Formeln Ia und Ib erhält man z.B. aus den entsprechenden 5-(N-3,4,5,6-Tetrahydrophthalimido)-zimtsäurechloriden VI durch Umsetzung mit einem Mercaptan VII oder einem Amin III.

Z.B.:

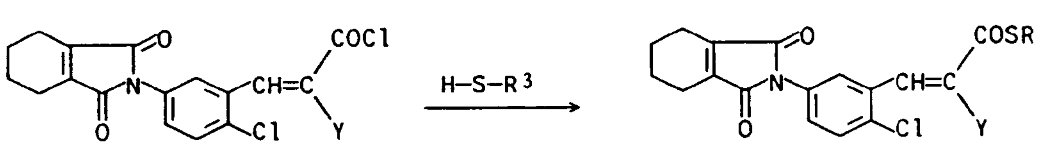


VI VII Ib

Die Reaktion wird in an sich bekannter Weise in der Regel in einem inerten organischen Lösungsmittel in Gegenwart einer Base bei Temperaturen von -15 bis 50°C, vorzugsweise bei -5 bis 30°C durchgeführt.

Als Lösungsmittel für diese Umsetzung eignen sich u.a. Kohlenwasserstoffe wie Toluol und Xylol, niedere Ester von Alkansäuren wie Essigsäureethylester und Ether wie Tetrahydrofuran, Dioxan und Diisopropylether.

Geeignete Hilfsbasen sind z.B. tertiäre Amine wie die Obengenannten sowie Pyridin, Collidin und Methylpiperidin, bevorzugt jedoch Triethylamin.

Eine Synthese für das Zimtsäurechlorid VI ist beispielsweise in EP-A-240 659 beschrieben worden.

Im Hinblick auf die bestimmungsgemäße Verwendung der Verbindungen Ia und/oder Ib kommen als Substituenten folgende Reste in Betracht:

Y Chlor; Brom;

Alkyl wie Methyl, Ethyl, n-Propyl, iso-Propyl, n-Butyl, iso-Butyl, sec.-Butyl und tert.-Butyl, vorzugsweise Methyl und Ethyl;

$R^1$ Wasserstoff; Phenyl; Benzyl;

Alkyl wie unter Y genannt sowie n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere Methyl und Ethyl,

Alkenyl wie Allyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Ethyl-2-propenyl, 2-Hexenyl, 3-Hexenyl, 4-Hexenyl, 5-Hexenyl, 1-Methyl-2-hexenyl, 2-Methyl-2-pentenyl, 3-Methyl-2-pentenyl, 4-Methyl-2-pentenyl, 5-Methyl-2-pentenyl, 1-Ethyl-2-butenyl, 2-Ethyl-2-butenyl, 1-Ethyl-1-methyl-2-propenyl und 1-Ethyl-2-methyl-2-propenyl, insbesondere Allyl,

Alkinyl wie Propin-2-yl, Butin-2-yl, Butin-3-yl, 1-Methylpropin-2-yl, Pentin-2-yl, Pentin-3-yl, Pentin-4-yl, 1-Methylbutin-2-yl, 1-Methylbutin-3-yl, 2-Methylbutin-3-yl, 1,1-Dimethylpropin-2-yl, 1-Ethylpropin-2-yl, Hexin-2-yl, Hexin-3-yl, Hexin-4-yl, Hexin-5-yl, 1-Methylpentin-2-yl, 4-Methylpentin-2-yl, 5-Methylpentin-2-yl, 1,1-Dimethylbutin-2-yl, 1-Ethylbutin-2-yl und 1-Ethyl-1-methylpropin-2-yl, insbesondere Propin-2-yl.

Unter den Resten $R^1$ können $C_2$-$C_4$-Alkylgruppen, Phenylrestes sowie Benzylreste ihrerseits folgende Substituenten tragen:

Hydroxyl;

Alkoxy wie Methoxy, Ethoxy, n-Propyloxy, iso-Propyloxy, n-Butyloxy, iso-Butyloxy und tert.-Butoxy in 2-, 3- und 4-Stellung des Akylrestes, insbesondere Methoxy, Ethoxy und iso-Propyloxy in 2-, 3- und 4-Stellung des Alkylrestes,

Alkylthio wie Methylthio, Ethylthio, n-Propylthio, iso-Propylthio, n-Butylthio, iso-Butylthio und tert.-Butylthio in 2-, 3- und 4-Stellung des Alkylrestes, insbesondere Methylthio und Ethylthio in 2-Stellung des Alkylrestes.

Bedeutet $R^1$ eine Phenyl- oder Benzylgruppe, kommen des weiteren folgende Substituenten in Betracht:

Cyano; Nitro; Halogenatome wie Fluor, Chlor und Brom, vorzugsweise Chlor; Alkyl, Alkoxy, Alkylthio und Halogenalkyl wie vorstehend genannt sowie

Alkylamino und Dialkylamino wie Methylamino, Dimethylamino, Ethylamino, Methylethylamino, Diethylamino, n-Propylamino, N-Methyl-N-propylamino, N-Ethyl-N-propylamino, N,N-Dipropylamino und N,N-Di-iso-propylamino, insbesondere N,N-Dimethylamino,

wobei $R^1$ nicht Wasserstoff bedeutet, wenn Y die Bedeutung Chlor und Brom hat.

$R^2$ $C_5$-$C_8$-Alkyl, wie bei $R^1$ genannt;

Alkoxy wie Methoxy, Ethoxy, n-Propyloxy, iso-Propyloxy sowie die isomeren Butoxy-, Pentyloxy- und Hexyloxygruppen, insbesondere Methoxy und Ethoxy;

Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 1-Methyl-2-butenyloxy, 2-Methyl-2-butenyloxy, 3-Methyl-2-butenyloxy und 1-Ethyl-2-propenyloxy, insbesondere 2-Propenyloxy und 2-Butenyloxy und

Alkinyloxy wie 2-Propinyloxy, 2-Butinyloxy, 3-Butinyloxy, 1-Methyl-2-propinyloxy, 2-Pentinyloxy, 1-Methyl-2-butinyloxy, 1,1-Dimethyl-2-propinyloxy, 1-Ethyl-2-propinyloxy und 2-Pentinyloxy, insbesondere 2-Propinyloxy, 2-Butinyloxy und 3-Butinyloxy.

$R^3$ steht für die unter $R^1$ genannten unsubstituierten $C_1$-$C_6$-Alkylgruppen sowie für eine der obengenannten $C_2$-$C_6$-Alkylgruppen, welche durch die bereits erwähnten Alkoxy- oder Alkylthioreste oder durch Hydroxy, vorzugsweise einfach substituiert sein können.

Beispiel für sehr aktive Verbindungen Ia und Ib sind in den nachstehenden Tabellen 1 und 2 aufgeführt.

Tabelle 1

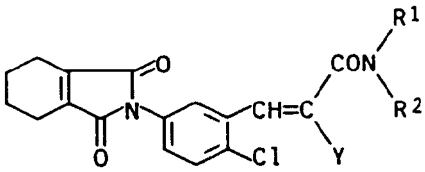

Ia

| Y | $R^1$ | $R^2$ |
|---|---|---|
| $CH_3$ | H | $(CH_2)_4CH_3$ |
| $CH_3$ | H | $CH_2CH(CH_3)(CH_2)_2CH_3$ |
| $CH_3$ | H | $(CH_2)_2CH(CH_3)_2$ |
| $CH_3$ | H | $CH_2CH(CH_2CH_3)_2$ |
| $CH_3$ | H | $CH(CH_3)CH(CH_3)_2$ |
| $CH_3$ | H | $CH_2C(CH_3)_3$ |
| $CH_3$ | H | $CH_2CH=CH_2$ |
| $CH_3$ | H | $CH_2C(CH_3)=CH_2$ |
| $CH_3$ | H | $CH_2CH=CHCH_3$ |
| $CH_3$ | H | $CH_2C\equiv CH$ |
| $CH_3$ | H | $CH(CH_3)C\equiv CH$ |
| $CH_3$ | H | $CH_2C\equiv CCH_3$ |
| $CH_3$ | H | $CH_2Ph$ |
| $CH_3$ | H | $(CH_2)_2OCH_3$ |
| $CH_3$ | H | $(CH_2)_2OCH_2CH_3$ |
| $CH_3$ | H | $CH_2CH(CH_3)OCH_3$ |
| $CH_3$ | H | $CH(CH_3)CH_2OCH_3$ |
| $CH_3$ | H | $CH(CH_2CH_3)CH_2OCH_3$ |
| $CH_3$ | H | $CH(CH_2OCH_3)(CH_2)_2OCH_3$ |
| $CH_3$ | H | $(CH_2)_2SCH_3$ |
| $CH_3$ | H | $(CH_2)_2SCH_2CH_3$ |
| $CH_3$ | H | $OCH_3$ |
| $CH_3$ | H | $OCH_2CH_3$ |
| $CH_3$ | H | $OCH_2CH=CH_2$ |
| $CH_3$ | H | $OCH_2C(CH_3)=CH_2$ |
| $CH_3$ | H | $OCH_2C\equiv CH$ |
| $CH_3$ | H | Ph |
| $CH_3$ | H | $2\text{-F-}C_6H_4$ |
| $CH_3$ | H | $2\text{-Cl-}C_6H_4$ |
| $CH_3$ | H | $2\text{-}CH_3\text{-}C_6H_4$ |
| $CH_3$ | H | $2\text{-}OCH_3\text{-}C_6H_4$ |
| $CH_3$ | H | $3\text{-Cl-}C_6H_4$ |
| $CH_3$ | H | $4\text{-Cl-}C_6H_4$ |
| $CH_3$ | H | $3\text{-}CH_3\text{-}C_6H_4$ |
| $CH_3$ | H | $4\text{-}OCH_3\text{-}C_6H_4$ |

Tabelle 1 (Fortsetzung)

| Y | $R^1$ | $R^2$ |
|---|---|---|
| $CH_3$ | H | 2,4-Cl,Cl-$C_6H_3$ |
| $CH_3$ | H | 2-Cl,4-$CH_3$-$C_6H_3$ |
| $CH_3$ | H | 2-Cl,6-$CH_3$-$C_6H_3$ |
| $CH_3$ | H | 3-$CF_3$-$C_6H_4$ |
| $CH_3$ | H | 4-CN-$C_6H_4$ |
| $CH_3$ | H | 3-$NO_2$-$C_6H_4$ |
| $CH_3$ | H | 2-$SCH_3$-$C_6H_4$ |
| $CH_3$ | H | 4-$N(CH_3)_2$-$C_6H_4$ |
| $CH_2CH_3$ | H | $(CH_2)_4CH_3$ |
| $CH_2CH_3$ | H | $(CH_2)_2CH(CH_3)_2$ |
| $CH_2CH_3$ | H | $CH_2CH=CH_2$ |
| $CH_2CH_3$ | H | $CH_2C\equiv CH$ |
| $CH_2CH_3$ | H | $CH_2Ph$ |
| $CH_2CH_3$ | H | Ph |
| $CH_2CH_3$ | H | 2-Cl-Ph |
| $CH_2CH_3$ | H | $(CH_2)_2OH$ |
| $CH_2CH_3$ | H | $(CH_2)_2OCH_3$ |
| $CH_2CH_3$ | H | $(CH_2)_2SCH_3$ |
| $CH_2CH_3$ | H | $OCH_3$ |
| $CH_2CH_3$ | H | $OCH_2CH=CH_2$ |
| $CH_2CH_3$ | H | $OCH_2C\equiv CH$ |
| Cl | $CH_3$ | $(CH_2)_4CH_3$ |
| Cl | $CH_3$ | $(CH_2)_2CH(CH_3)_2$ |
| Cl | $CH_3$ | $CH_2CH=CH_2$ |
| Cl | $CH_3$ | $CH_2C\equiv CH$ |
| Cl | $CH_3$ | $CH_2Ph$ |
| Cl | $CH_3$ | Ph |
| Cl | $CH_3$ | 2-Cl-Ph |
| Cl | $CH_3$ | $(CH_2)_2OH$ |
| Cl | $CH_3$ | $(CH_2)_2OCH_3$ |
| Cl | $CH_3$ | $(CH_2)_2SCH_3$ |
| Cl | $CH_3$ | $OCH_3$ |
| Cl | $CH_3$ | $OCH_2CH=CH_2$ |
| Cl | $CH_3$ | $OCH_2C\equiv CH$ |
| Br | $CH_3$ | $(CH_2)_4CH_3$ |
| Br | $CH_3$ | $(CH_2)_2CH(CH_3)_2$ |
| Br | $CH_3$ | $CH_2CH=CH_2$ |
| Br | $CH_3$ | $CH_2C\equiv CH$ |
| Br | $CH_3$ | $CH_2Ph$ |

Tabelle 1 (Fortsetzung)

| Y | $R^1$ | $R^2$ |
|---|---|---|
| Br | $CH_3$ | Ph |
| Br | $CH_3$ | 2-Cl-Ph |
| Br | $CH_3$ | $(CH_2)_2OH$ |
| Br | $CH_3$ | $(CH_2)_2OCH_3$ |
| Br | $CH_3$ | $(CH_2)_2SCH_3$ |
| Br | $CH_3$ | $OCH_3$ |
| Br | $CH_3$ | $OCH_2CH=CH_2$ |
| Br | $CH_3$ | $OCH_2C\equiv CH$ |
| $CH_3$ | $CH_3$ | $(CH_2)_4CH_3$ |
| $CH_3$ | $CH_3$ | $(CH_2)_2CH(CH_3)_2$ |
| $CH_3$ | $CH_3$ | $CH_2CH=CH_2$ |
| $CH_3$ | $CH_3$ | $CH_2C\equiv CH$ |
| $CH_3$ | $CH_3$ | $CH_2Ph$ |
| $CH_3$ | $CH_3$ | Ph |
| $CH_3$ | $CH_3$ | 2-Cl-Ph |
| $CH_3$ | $CH_3$ | $(CH_2)_2OH$ |
| $CH_3$ | $CH_3$ | $(CH_2)_2OCH_3$ |
| $CH_3$ | $CH_3$ | $(CH_2)_2SCH_3$ |
| $CH_3$ | $CH_3$ | $OCH_3$ |
| $CH_3$ | $CH_3$ | $OCH_2CH=CH_2$ |
| $CH_3$ | $CH_3$ | $OCH_2C\equiv CH$ |
| $CH_3$ | $CH_2CH_3$ | $(CH_2)_4CH_3$ |
| $CH_3$ | $CH_2CH_3$ | $(CH_2)_2CH(CH_3)_2$ |
| $CH_3$ | $CH_2CH_3$ | $CH_2CH=CH_2$ |
| $CH_3$ | $CH_2CH_3$ | $CH_2C\equiv CH$ |
| $CH_3$ | $CH_2CH_3$ | $CH_2Ph$ |
| $CH_3$ | $CH_2CH_3$ | Ph |
| $CH_3$ | $CH_2CH_3$ | 2-Cl-Ph |
| $CH_3$ | $CH_2CH_3$ | $(CH_2)_2OH$ |
| $CH_3$ | $CH_2CH_3$ | $(CH_2)_2OCH_3$ |
| $CH_3$ | $CH_2CH_3$ | $(CH_2)_2SCH_3$ |
| $CH_3$ | $CH_2CH_3$ | $OCH_3$ |
| $CH_3$ | $CH_2CH_3$ | $OCH_2CH=CH_2$ |
| $CH_3$ | $CH_2CH_3$ | $OCH_2C\equiv CH$ |
| $CH_3$ | $CH(CH_3)_2$ | $(CH_2)_4CH_3$ |
| $CH_3$ | $CH(CH_3)_2$ | $(CH_2)_2CH(CH_3)_2$ |
| $CH_3$ | $CH(CH_3)_2$ | $CH_2CH=CH_2$ |
| $CH_3$ | $CH(CH_3)_2$ | $CH_2C\equiv CH$ |
| $CH_3$ | $CH(CH_3)_2$ | $CH_2Ph$ |

Tabelle 1 (Fortsetzung)

| Y | $R^1$ | $R^2$ |
|---|---|---|
| $CH_3$ | $CH(CH_3)_2$ | Ph |
| $CH_3$ | $CH(CH_3)_2$ | 2-Cl-Ph |
| $CH_3$ | $CH(CH_3)_2$ | $(CH_2)_2OH$ |
| $CH_3$ | $CH(CH_3)_2$ | $(CH_2)_2OCH_3$ |
| $CH_3$ | $CH(CH_3)_2$ | $(CH_2)_2SCH_3$ |
| $CH_3$ | $CH(CH_3)_2$ | $OCH_3$ |
| $CH_3$ | $CH(CH_3)_2$ | $OCH_2CH=CH_2$ |
| $CH_3$ | $CH(CH_3)_2$ | $OCH_2C\equiv CH$ |
| $CH_3$ | $CH_2CH=CH_2$ | $(CH_2)_4CH_3$ |
| $CH_3$ | $CH_2CH=CH_2$ | $(CH_2)_2CH(CH_3)_2$ |
| $CH_3$ | $CH_2CH=CH_2$ | $CH_2CH=CH_2$ |
| $CH_3$ | $CH_2CH=CH_2$ | $CH_2C\equiv CH$ |
| $CH_3$ | $CH_2CH=CH_2$ | $CH_2Ph$ |
| $CH_3$ | $CH_2CH=CH_2$ | Ph |
| $CH_3$ | $CH_2CH=CH_2$ | 2-Cl-Ph |
| $CH_3$ | $CH_2CH=CH_2$ | $(CH_2)_2OH$ |
| $CH_3$ | $CH_2CH=CH_2$ | $(CH_2)_2OCH_3$ |
| $CH_3$ | $CH_2CH=CH_2$ | $(CH_2)_2SCH_3$ |
| $CH_3$ | $CH_2CH=CH_2$ | $OCH_3$ |
| $CH_3$ | $CH_2CH=CH_2$ | $OCH_2CH=CH_2$ |
| $CH_3$ | $CH_2CH=CH_2$ | $OCH_2C\equiv CH$ |
| $CH_3$ | $CH_2C\equiv CH$ | $(CH_2)_4CH_3$ |
| $CH_3$ | $CH_2C\equiv CH$ | $(CH_2)_2CH(CH_3)_2$ |
| $CH_3$ | $CH_2C\equiv CH$ | $CH_2CH=CH_2$ |
| $CH_3$ | $CH_2C\equiv CH$ | $CH_2C\equiv CH$ |
| $CH_3$ | $CH_2C\equiv CH$ | $CH_2Ph$ |
| $CH_3$ | $CH_2C\equiv CH$ | Ph |
| $CH_3$ | $CH_2C\equiv CH$ | 2-Cl-Ph |
| $CH_3$ | $CH_2C\equiv CH$ | $(CH_2)_2OH$ |
| $CH_3$ | $CH_2C\equiv CH$ | $(CH_2)_2OCH_3$ |
| $CH_3$ | $CH_2C\equiv CH$ | $(CH_2)_2SCH_3$ |
| $CH_3$ | $CH_2C\equiv CH$ | $OCH_3$ |
| $CH_3$ | $CH_2C\equiv CH$ | $OCH_2CH=CH_2$ |
| $CH_3$ | $CH_2C\equiv CH$ | $CH_2C\equiv CH$ |
| $CH_3$ | $(CH_2)_2OCH_3$ | $(CH_2)_4CH_3$ |
| $CH_3$ | $(CH_2)_2OCH_3$ | $(CH_2)_2CH(CH_3)_2$ |
| $CH_3$ | $(CH_2)_2OCH_3$ | $CH_2CH=CH_2$ |
| $CH_3$ | $(CH_2)_2OCH_3$ | $CH_2C\equiv CH$ |
| $CH_3$ | $(CH_2)_2OCH_3$ | $CH_2Ph$ |

Tabelle 1 (Fortsetzung)

| Y | $R^1$ | $R^2$ |
|---|---|---|
| $CH_3$ | $(CH_2)_2OCH_3$ | Ph |
| $CH_3$ | $(CH_2)_2OCH_3$ | 2-Cl-Ph |
| $CH_3$ | $(CH_2)_2OCH_3$ | $(CH_2)_2OH$ |
| $CH_3$ | $(CH_2)_2OCH_3$ | $(CH_2)_2OCH_3$ |
| $CH_3$ | $(CH_2)_2OCH_3$ | $(CH_2)_2SCH_3$ |
| $CH_3$ | $(CH_2)_2OCH_3$ | $OCH_3$ |
| $CH_3$ | $(CH_2)_2OCH_3$ | $OCH_2CH=CH_2$ |
| $CH_3$ | $(CH_2)_2OCH_3$ | $OCH_2C\equiv CH$ |

Tabelle 2

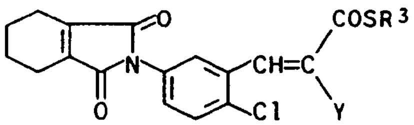

Ib

| Y | $R^3$ |
|---|---|
| $CH_3$ | $CH_3$ |
| $CH_3$ | $CH_2CH_3$ |
| $CH_3$ | $(CH_2)_2CH_3$ |
| $CH_3$ | $CH(CH_3)_2$ |
| $CH_3$ | $CH_2CH(CH_3)_2$ |
| $CH_3$ | $C(CH_3)_3$ |
| $CH_3$ | $(CH_2)_2OCH_3$ |
| $CH_3$ | $(CH_2)_2SCH_3$ |
| $CH_3$ | $(CH_2)_2OH$ |
| $CH_2CH_3$ | $CH_3$ |
| $CH_2CH_3$ | $CH_2CH_3$ |
| $CH_2CH_3$ | $(CH_2)_2CH_3$ |
| $CH_2CH_3$ | $CH(CH_3)_2$ |
| $CH_2CH_3$ | $CH_2CH(CH_3)_2$ |
| $CH_2CH_3$ | $C(CH_3)_3$ |
| $CH_2CH_3$ | $(CH_2)_2OCH_3$ |
| $CH_2CH_3$ | $(CH_2)_2SCH_3$ |
| $CH_2CH_3$ | $(CH_2)_2OH$ |
| Cl | $CH_3$ |
| Cl | $CH_2CH_3$ |
| Cl | $(CH_2)_2CH_3$ |
| Cl | $CH(CH_3)_2$ |
| Cl | $CH_2CH(CH_3)_2$ |
| Cl | $C(CH_3)_3$ |
| Cl | $(CH_2)_2OCH_3$ |
| Cl | $(CH_2)_2SCH_3$ |
| Cl | $(CH_2)_2OH$ |
| Br | $CH_3$ |
| Br | $CH_2CH_3$ |
| Br | $(CH_2)_2CH_3$ |
| Br | $CH(CH_3)_2$ |
| Br | $CH_2CH(CH_3)_2$ |
| Br | $C(CH_3)_3$ |
| Br | $(CH_2)_2OCH_3$ |
| Br | $(CH_2)_2SCH_3$ |
| Br | $(CH_2)_2OH$ |

Die 5-(N-3,4,5,6-Tetrahydrophthalimido)-zimtsäurederivate Ia und Ib bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen Ia und Ib eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,1 und 95 Gew.%, vorzugsweise zwischen 0,5 und 90 Gew.%, Wirkstoff.

Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen Ia und Ib können beispielsweise wie folgt formuliert werden:

I. Man vermischt 90 Gewichtsteile der Verbindung Nr. 1.001 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.008 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.003 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 2.010 werden in einer Mischung gelöst, die aus 25 Gewichtsteilen Cyclohexanon, 65 Gewichsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.001 werden mit 3 Gewichtsteilen des Natriumsalzes der Diisobu-

tylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 2.007 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1.015 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.006 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (postdirected, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha.

In Anbetracht der Vielseitigkeit der Applikationsmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |

| Botanischer Name | Deutscher Name |
|---|---|
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |

| Botanischer Name | Deutscher Name |
|---|---|
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die 5-(N-3,4,5,6-Tetrahydrophthalimido)zimtsäurederivate der Formel Ia und Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die neuen Verbindungen der Formel Ia und Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen Ia und Ib benutzt. Die so erhaltenen Verbindungen sind in den nachstehenden Tabellen mit physikalischen Angaben aufgeführt.

Beispiel 1

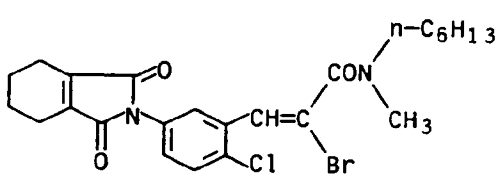

a) Eine Lösung von 12,3 g (0,05 mol) 2-Chlor-5-nitrozimtsäurechlorid in 40 ml Tetrahyhdrofuran wurde bei 0 bis 5°C mit 11,5 g (0,10 mol) N-Methylhexylamin in 100 ml Tetrahydrofuran versetzt. Nach 2 Stunden Rühren wurde das Lösungsmittel entfernt, der so erhaltene Rückstand in Essigsäureethylester gelöst und mit 5 %iger Natronlauge extrahiert. Nach Waschen der organischen Phase mit Wasser, Abtrennen und Trocknen erhielt man 13,3 g (81 % d.Th.) 2-Chlor-5-nitro-zimtsäure-N-methyl-N-hexylamid.

b) 120 g (0,037 mol) der unter a) erhaltenen Verbindung in 100 ml 1,1,1-Trichlorethan wurden bei 25°C mit 6,4 g (0,04 mol) Brom in 10 ml 1,1,1-Trichlorethan versetzt. Nach 1 Stunde Rühren bei 40°C wurden weitere 0,5 ml Brom zugesetzt und wiederum 1 Stunde bei 40°C belassen. Nach dem Abdestillieren des Lösungsmittels wurde der Rückstand mit Petrolether verrieben.

Die so erhaltenen Kristalle wurden in 150 ml Methanol gelöst und bei 25°C tropfenweise mit 8,0 g (0,045 mol) 30 %iger Natriummethylat-Lösung in 80 ml Methanol versetzt. Nach 2 Stunden bei 65°C wurde das Lösungsmittel entfernt, der Rückstand mit Wasser verrührt und mit Essigsäureethylester extrahiert. Nach Waschen, Trocnen und Einengen der organischen Phase erhielt man 11,8 g (79 %) rohes 2-Chlor-5-nitro-α-bromzimtsäure-N-methyl-N-hexylamid, welches ohne weitere Reinigung umgesetzt wurde.

c) Zu einer Suspension von 8,1 g (0,145 mol) Eisenpulver in einer auf 60°C erwärmten Mischung von 80 ml Methanol und 40 ml Eisessig gab man portionsweise 11,0 g (0,049 mol) der unter b) erhaltenen Nitrover bindung und erhitzte anschließend 2 Stunden auf 80°C. Nach erfolgter Umsetzung wurde in Wasser eingerührt, vom Feststoff abfiltriert und die wäßrige Phase mit Essigsäureethylester extrahiert. Nach dem Waschen der organischen Phase mit Wasser, Trocknen und Einengen erhielt man 10,2 g (100 %) 2-Chlor-5-amino-α-bromzimtsäure-N-methyl-N-hexylamid als Öl, welches ohne weitere Reingiung umgesetzt wurde.

d) Eine Mischung aus 10,2 g (0,027 mol) des unter c) erhaltenen Anilins und 4,1 g (0,027 mol) Tetrahydrophthalsäureanhydrid in 80 ml Eisessig wurde 3 Stunden auf Siedetemperatur erhitzt. Nach erfolgter Umsetzung wurde die Reaktionsmischung in Wasser gerührt und mit Essigsäureethylester extrahiert. Aus der organischen Phase erhielt man nach Waschen, Trocknen und Einengen 7,1 g (52 % d.Th.) 5-(N-3,4,5,6-Tetrahydrophthalimido)-2-chlor-α-bromzimtsäure-N′-methyl-N′-hexylamid (Wirkstoffbeispiel 1.028) als Öl.

Beispiel 2

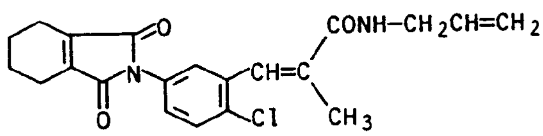

a) 4,6 g (0,08 mol) Alkylamin in 50 ml Tetrahydrofuran wurden bei 5 bis 10°C mit 10,4 g (0,04 mol) 2-Chlor-5-nitro-α-methylzimtsäurechlorid in 100 ml Tetrahydrofuran versetzt. Nach 2 Stunden wurde das Lösungsmittel entfernt, in Wasser eingerührt und der dabei entstehende Feststoff isoliert. Nach dem Trocknen des Feststoffs erhielt man 8,1 g (81 % d.Th.) 2-Chlor-5-nitro-α-methylzimtsäure-N-allylamid vom Fp. 111 bis 112°C.

b) Analog zu 1c) erhielt man aus 9,1 g (0,032 mol) der unter a) gewonnenen Nitroverbindung mit 9,1 g (0,162 mol) Eisenpulver in 150 ml Methanol und 100 ml Eisessig nach der Aufarbeitung 8,1 g (100 % d.Th.) 5-Amino-2-chlor-α-methylzimtsäure-N-allylamid als Öl.

c) Analog zu 1d) erhielt man aus 8,1 g (0,032 mol) des unter b) erhaltenen Amins und 4,9 g (0,032 mol) Tetrahydrophthalimidsäureanhydrid in 100 ml Eisessig nach der Aufarbeitung 9,2 g (74 % d.Th.) 5-(N-3,4,5,6-Tetrahydrophthalimido)-2-chlor-α-methylzimtsäure-N-allylamid vom Fp. 89 bis 90°C (Wirkstoffbeispiel 1.007).

Beispiel 3

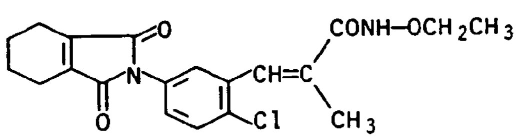

a) 10,8 g (0,06 mol) 30 %ige Natriummethylatlösung in Methanol wurden bei 25°C mit 5,9 g (0,06 mol) O-Ethylhydroxylamin-hydrochlorid in 100 ml Tetrahydrofuran und anschließend bei 0 bis 5°C mit 10,9 g (0,03 mol) 5-(N-3,4,5,6-Tetrahydrophthalimido)-2-chlor-α-methylzimtsäurechlorid in 50 ml Tetrahydrofuran versetzt. Nach 4 Stunden bei 25°C wurde die Reaktionsmischung eingeengt, der Rücktand mit Wasser verrührt und mit Essigsäureethylester extrahiert. Aus der organischen Phase erhielt man nach Waschen, Trocknen, Einengen und Verrühren des Rückstandes mit Petrolether (10,0 g (86 % d.Th.) 5-(N-3,4,5,6-Tetrahydrophthalimido)-2-chlor-α-methylzimtsäure-N'-ethoxyamid vom Fp. 83 bis 84°C (Wirkstoffbeispiel 1.016).

Beispiel 4

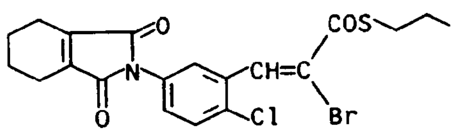

a) Eine Mischung aus 330 ml Wasser, 170 ml Methanol und 40 g (1 mol) Natriumhydroxid wurden bei 65°C mit 120,3 g (0,28 mol) 5-(N-3,4,5,6-Tetrahydrophthalsäureimido)-2-chlor-α-bromzimtsäuremethylester (EP 240 659) versetzt und zum Sieden erhitzt. Nach 7 Minuten ließ man auf 40°C abkühlen, gab in 250 ml 10 %iger Salzsäure und extrahierte zweimal mit 250 ml Essigsäureethylester. Die organische Phase wurde abgetrennt, getrocknet und das Lösungsmittel bei vermindertem Druck abgezogen. Nach Kristallisieren des öligen Rückstands mit Eisessig/Wasser und Trocknen erhielt man 5-(N-3,4,5,6-Tetrahydrophthalimido)-2-chlor-α-bromzimtsäure. Fp. 90°C.

b) 118 g (0,28 mol) der bei a) hergestellten Säure wurden in 400 ml Toluol gelöst, mit 6 Tropfen Dimethylformamid versetzt und auf 80°C erwärmt. Anschließend wurden 107 g (0,9 mol) Thionylchlorid zugegeben und 5 Stunden zum Sieden erhitzt. Nach dem Abdestillieren der Lösungsmittel bei vermindertem Druck erhielt man 89 g rohes 5-(N-3,4,5,6-Tetrahydrophthalimido)-2-chlor-α-bromzimtsäurechlorid als Öl.

c) Eine Lösung aus 2,3 g (0,03 mol) n-Propylmoercaptan und 3,5 g (0,035 mol) Triethylamin in 50 ml Essigsäureethylester wurde bei 25 bis 30°C mit 12,9 g (0,03 mol) des bei b) hergestellten Säurechlorids in 150 ml Essigsäureethylester versetzt, 3 Stunden bei 25 bis 30°C gerührt und anschließend mit 50 ml 5 %iger Natronlauge extrahiert. Nach dem Waschen der organischen Phase mit Wasser, Trocknen und Abdestillieren des Lösungsmittels erhielt man 10,3 g 5-(N-3,4,5,6-Tetrahydrophthalimido)-2-chlor-α-bromzimtsäure-n-propylthioester als Öl (Wirkstoffbeispiel 2.003, Tabelle 4).

Tabelle 3

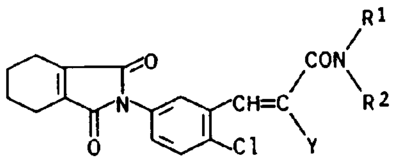

| Nr. | Y | R1 | R2 | Fp °C |
|---|---|---|---|---|
| 1.001 | $CH_3$ | H | 3-Methyl-butyl-2 | 122-123 |
| 1.002 | $CH_3$ | H | Neopentyl | 123-124 |
| 1.003 | $CH_3$ | H | Isoamyl | 88- 89 |
| 1.004 | $CH_3$ | H | 2-Ethylbutyl | 117-118 |
| 1.005 | $CH_3$ | H | n-Pentyl | 87- 88 |
| 1.006 | $CH_3$ | H | 2-Methylpentyl | 68- 69 |
| 1.007 | $CH_3$ | H | Allyl | 89- 90 |
| 1.008 | $CH_3$ | H | 2-Methylallyl | 70- 71 |
| 1.009 | $C_2H_5$ | H | Allyl | 117-118 |
| 1.010 | $CH_3$ | H | 2-Methoxyethyl | 99-100 |
| 1.011 | $CH_3$ | H | 2-Methyl-thioethyl | öl |
| 1.012 | $CH_3$ | H | 3-Methoxypropyl-2 | 70- 71 |
| 1.013 | $CH_3$ | H | 1-Methoxybutyl-2 | 98- 99 |
| 1.014 | $CH_3$ | H | 4-Methoxybutyl-2 | öl |
| 1.015 | $CH_3$ | $CH_2CH_2OCH_3$ | $CH_2CH_2OCH_3$ | 59- 61 |
| 1.016 | $CH_3$ | H | Ethoxy | 83- 84 |
| 1.017 | $CH_3$ | H | Allyloxy | 120-121 |
| 1.018 | $CH_3$ | $CH_3$ | Methoxy | 99-101 |
| 1.019 | $C_2H_5$ | H | Phenyl | 179-180 |
| 1.020 | $CH_3$ | H | 2-Cl-phenyl | 159-160 |
| 1.021 | $CH_3$ | H | 3-Cl-phenyl | 176-178 |
| 1.022 | $CH_3$ | H | 4-Cl-phenyl | 128-130 |
| 1.023 | $CH_3$ | H | 2-$CH_3$-phenyl | 104-105 |
| 1.024 | $CH_3$ | H | 3-$CH_3$-phenyl | 172-173 |
| 1.025 | $CH_3$ | H | 2-$CH_3O$-phenyl | 204-205 |
| 1.026 | $CH_3$ | H | 2-Cl,6-$CH_3$-phenyl | 167-168 |
| 1.027 | $CH_3$ | H | 2,6-Cl,Cl-phenyl | 186-187 |
| 1.028 | Br | $CH_3$ | n-Hexyl | öl |

Tabelle 4

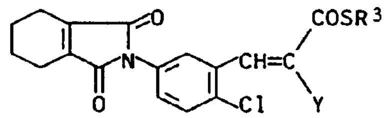

| Nr. | Y | $R^3$ | Fp (°C) |
|---|---|---|---|
| 2.001 | $CH_3$ | $CH_3$ | 135-136 |
| 2.002 | $CH_3$ | $C_2H_5$ | 105-106 |
| 2.003 | $CH_3$ | $n-C_3H_7$ | 80- 82 |
| 2.004 | $CH_3$ | $n-C_4H_9$ | 75- 77 |
| 2.005 | $CH_3$ | $i-C_4H_9$ | 86- 87 |
| 2.006 | $CH_3$ | $CH_2CH_2OH$ | 77- 79 |
| 2.007 | Br | $CH_3$ | öl |
| 2.008 | Br | $C_2H_5$ | öl |
| 2.009 | Br | $n-C_3H_7$ | öl |
| 2.010 | Br | $i-C_3H_7$ | öl |
| 2.011 | Br | $CH_2CH_2OH$ | 70- 72 |
| 2.012 | Br | $CH_2CH_2OCH_3$ | öl |

Anwendungsbeispiele

Die Wirkung der 5-(N-3,4,5,6-Tetrahydrophthalimido)-zimtsäurederivate Ia und Ib auf das Wachstum der Testpflanzen ließ sich durch folgende Gewächshausversuche zeigen.

Als Kulturgefäße dienen Plastikblumentöpfe mit 300 $cm^3$ Inhalt und lehmigem Sand mit etwa 3,0 % Humus als Substrat. Die Samen der Testpflanzen wurden nach Arten getrennt eingesät.

Zum Zweck der Nachauflaufbehandlung werden entweder direkt gesäte oder in gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmengen für die Nachauflaufbehandlung beträgt 0,015 bis 0,03 kg/ha a.S.

Die Versuchsgefäße werden im Gewächshaus aufgestellt, wobei für wärmeliebende Arten wärmere Bereiche (20 bis 35°C) und für solche gemäßigter Klimate 10 bis 20°C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Mit 0,015 bzw. 0,03 kg/ha a.S. im Nachauflaufverfahren eingesetzt, lassen sich mit den Beispielen 1.005 und 2.002 breitblättrige unerwünschte Pflanzen sehr gut bekämpfen.

Die Verbindungen 1.006 und 1.008 bekämpfen bei Nachauflaufanwendung von 0,03 kg/ha a.S. unerwünschte breitblättrige Pflanzen sehr gut ohne Weizen als Beispielkultur nennenswert zu schädigen.

Die in den Gewächshausversuchen verwendeten Pflanzen setzen sich aus folgenden Arten zusammen:

| Lateinischer Name | Deutscher Name |
|---|---|
| Abutilon theophrasti | Chinesischer Hanf |
| Amaranthus retroflexus | Zurückgekrümmter Fuchsschwanz |
| Chrysanthemum corinarium | Kronenwucherblume |
| Euphorbia heterophylla | Wolfsmilchart |
| Galium aparine | Klettenlabkraut |
| Lamium amplexicaule | Stengelumfassene Traubnessel |
| Malva neglecta | Wegmalve |
| Mercurialis annua | einjähriges Bindelkraut |
| Oryza sativa | Reis |
| Solanum nigrum | Schwarzer Nachtschatten |
| Triticum aestivum | Sommerweizen |

## Patentansprüche

**Patentansprüche für folgende Vertragsstraaten : BE, CH, DE, FR, GB, IT, LI, NL**

1. 5-(N-3,4,5,6-Tetrahydrophthalimido)-zimtsäurederivate der Formeln Ia und Ib

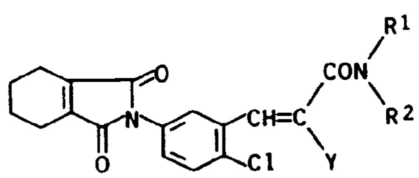

Ia

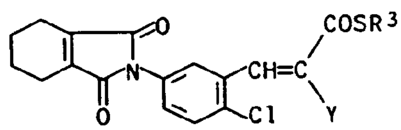

Ib

in denen die Substituenten folgende Bedeutung haben:

Y Chlor, Brom oder eine $C_1$-$C_4$-Alkylgruppe

$R^1$ Wasserstoff,

eine $C_1$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_3$-$C_6$-Alkenylgruppe,

eine $C_3$-$C_6$-Alkinylgruppe,

ein Phenylring oder ein Benzylrest, wobei die Ringe ein bis drei der folgenden Rest tragen können: Halogen, $C_1$-$C_4$-Alkyl, $C_1$-$C_4$-Alkoxy, $C_1$-$C_4$-Halogenalkyl, Cyano, Nitro, $C_1$-$C_4$-Halogenalkoxy, N-$C_1$-$C_4$-Alkylamino, N,N-Di-$C_1$-$C_4$-Alkylamino und/oder $C_1$-$C_4$-Alkylthio,

$R^2$ eine $C_5$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_1$-$C_6$-Alkoxygruppe,

eine $C_3$-$C_6$-Alkenylgruppe,

eine $C_3$-$C_6$-Alkenyloxygruppe,

eine $C_3$-$C_6$-Alkinylgruppe,

eine $C_3$-$C_6$-Alkinyloxygruppe,

eine Phenyl-, Phenoxy-, Benzyl- oder Benzyloxyrest, wobei diese Reste ein bis drei der folgenden Substituenten tragen können: Halogen, $C_1$-$C_4$-Alkyl, $C_1$-$C_4$-Alkoxy,

$C_1$-$C_4$-Alkylthio, $C_1$-$C_4$-Halogenalkyl, Cyano, Nitro, $C_1$-$C_4$-Halogenalkoxy, N-$C_1$-$C_4$-Alkylamino und/oder N,N-Di-$C_1$-$C_4$-alkylamino,

$R^3$ eine $C_1$-$C_6$-Alkylgruppe,

eine $C_2$-$C_6$-Alkylgruppe, welche ein bis drei der folgenden Substituenten tragen kann: $C_1$-$C_4$-Alkoxy, $C_1$-$C_4$-Alkylthio und/oder Hydroxy,
wobei $R^1$ nicht Wasserstoff bedeutet, wenn Y die Bedeutung Chlor und Brom hat.

2. Verfahren zur Herstellung der Verbindungen Ia gemäß Anspruch 1, worin Y $C_1$-$C_4$-Alkyl bedeutet, dadurch gekennzeichnet, daß man ein Nitrozimtsäurechlorid der Formel II

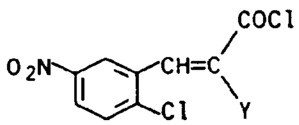
II

in an sich bekannter Weise in einem inerten organischen Lösungsmittel in Anwesenheit einer Base mit einem Amin der Formel III

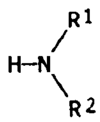
III

zum Nitrozimtsäureamid IV umsetzt,

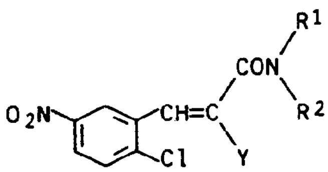
IV

anschließend zur Aminoverbindung V reduziert

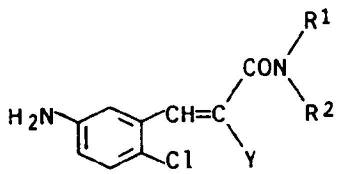
V

und V mit 3,4,5,6-Tetrahydrophthalsäureanhydrid kondensiert.

3. Verfahren zur Herstellung der Verbindungen Ia gemäß Anspruch 1, worin Y Chlor oder Brom bedeutet, dadurch gekennzeichnet, daß man ein Nitrozimtsäureamid der Formel IVa

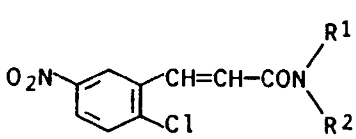
IVa

in einem inerten organischen Lösungsmittel mit Chlor oder Brom zum Zimtsäureamid der Formel IV

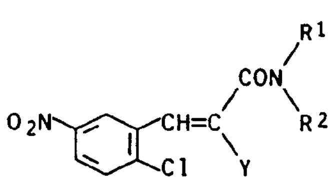
IV

halogeniert, anschließend reduziert (V) und mit 3,4,5,6-Tetrahydrophthalsäureanhydrid kondensiert.

4. Verfahren zur Herstellung der Verbindungen Ia und Ib gemäß Anspruch 1, dadurch gekennzeichnet, daß man ein 5-(N-3,4,5,6-Tetrahydrophthalimido)-zimtsäurechlorid der Formel VI

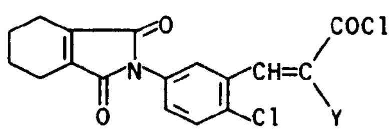

VI

in an sich bekannter Weise in einem inerten organischen Lösungsmittel entweder mit einem Mercaptan der Formel VII

$$H\text{-}S\text{-}R^3 \qquad VII$$

oder mit einem Amin der Formel III umsetzt.

5. Herbizide Mittel, enthaltend ein 5-(N-3,4,5,6-Tetrahydrophthalimido)-zimtsäurederivat der Formeln Ia und/oder Ib gemäß Anspruch 1 und inerte Zusatzstoffe.

6. Herbizide Mittel gemäß Anspruch 5, enthaltend ein 5-(N-3,4,5,6-Tetrahydrophthalimido)-zimtsäurederivat der Formeln Ia und/oder Ib und weitere wirksame Bestandteile.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines 5-(N-3,4,5,6-Tetrahydrophthalimido)-zimtsäurederivates Ia und/oder Ib gemäß Anspruch 1 behandelt.

8. Verwendung der 5-(N-3,4,5,6-Tetrahydrophthalimido)-zimtsäurederivate der Formel Ia und Ib gemäß Anspruch 1 als Herbizide.

**Patentansprüche für folgenden Vertragsstaat : ES**

1. Verfahren zur Herstellung von 5-(N-3,4,5,6-Tetrahydrophthalimido)-zimtsäurederivaten der Formel Ia

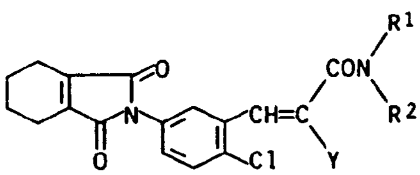

Ia

in denen die Substituenten folgende Bedeutung haben:

Y eine $C_1$-$C_4$-Alkylgruppe

$R^1$ Wasserstoff,

eine $C_1$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_3$-$C_6$-Alkenylgruppe,

eine $C_3$-$C_6$-Alkinylgruppe,

ein Phenylring oder ein Benzylrest, wobei die Ringe ein bis drei der folgenden Reste tragen können: Halogen, $C_1$-$C_4$-Alkyl, $C_1$-$C_4$-Alkoxy, $C_1$-$C_4$-Halogenalkyl, Cyano, Nitro, $C_1$-$C_4$-Halogenalkoxy, N-$C_1$-$C_4$-Alkylamino, N,N-Di-$C_1$-$C_4$-Alkylamino und/oder $C_1$-$C_4$-Alkylthio,

$R^2$ eine $C_5$-$C_8$-Alkylgruppe,

eine $C_2$-$C_4$-Alkylgruppe, welche einen Hydroxy-, $C_1$-$C_4$-Alkoxy- oder $C_1$-$C_4$-Alkylthiorest trägt,

eine $C_1$-$C_6$-Alkoxygruppe,

eine $C_3$-$C_6$-Alkenylgruppe,

eine $C_3$-$C_6$-Alkenyloxygruppe,

eine $C_3$-$C_6$-Alkinylgruppe,

eine $C_3$-$C_6$-Alkinyloxygruppe,

eine Phenyl-, Phenoxy-, Benzyl- oder Benzyloxyrest, wobei diese Reste ein bis drei der folgenden Substituenten tragen können: Halogen, $C_1$-$C_4$-Alkyl, $C_1$-$C_4$-Alkoxy,

$C_1$-$C_4$-Alkylthio, $C_1$-$C_4$-Halogenalkyl, Cyano, Nitro, $C_1$-$C_4$-Halogenalkoxy, N-$C_1$-$C_4$-Alkylamino und/oder N,N-Di-$C_1$-$C_4$-alkylamino,

dadurch gekennzeichnet, daß man ein Nitrozimtsäurechlorid der Formel II

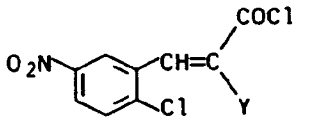

II

in an sich bekannter Weise in einem inerten organischen Lösungsmittel in Anwesenheit einer Base mit einem Amin der Formel III

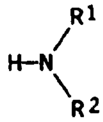

III

zum Nitrozimtsäureamid IV umsetzt,

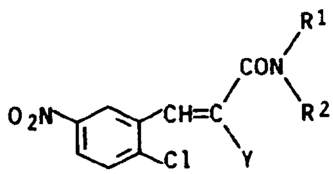

IV

anschließend zur Aminoverbindung V reduziert

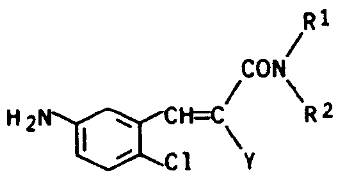

V

und V mit 3,4,5,6-Tetrahydrophthalsäureanhydrid kondensiert.

2. Verfahren zur Herstellung der Verbindungen Ia gemäß Anspruch 1, worin Y Chlor oder Brom und $R^1$ nicht Wasserstoff bedeutet, dadurch gekennzeichnet, daß man ein Nitrozimtsäureamid der Formel IVa

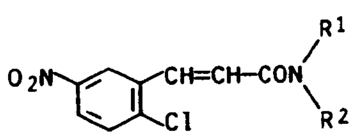

IVa

in einem inerten organischen Lösungsmittel mit Chlor oder Brom zum Zimtsäureamid der Formel IV

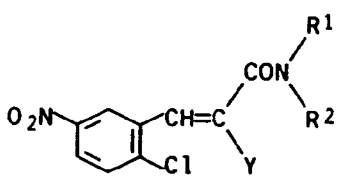

IV

halogeniert, anschließend reduziert (V) und mit 3,4,5,6-Tetrahydrophthalsäureanhydrid kondensiert.

3. Verfahren zur Herstellung der Verbindungen Ia gemäß den Ansprüchen 1 und 2 und Ib

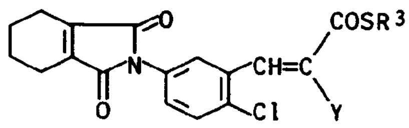

Ib

in der die Substituenten folgende Bedeutung haben:

Y Chlor, Brom oder eine $C_1$-$C_4$-Alkylgruppe,

$R^3$ eine $C_1$-$C_6$-Alkylgruppe,

eine $C_2$-$C_6$-Alkylgruppe, welche ein bis drei der folgenden Substituenten tragen kann: $C_1$-$C_4$-Alkoxy, $C_1$-$C_4$-Alkylthio und/oder Hydroxy,

dadurch gekenzeichnet, daß man ein 5-(N-3,4,5,6-Tetrahydrophthalimido)-zimtsäurechlorid der Formel VI

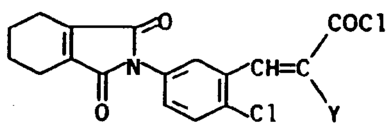

VI

in an sich bekannter Weise in einem inerten organischen Lösungsmittel entweder mit einem Mercaptan der Formel VII

$$H\text{-}S\text{-}R^3 \qquad VII$$

oder mit einem Amin der Formel III umsetzt.

4. Herbizide Mittel, enthaltend ein 5-(N-3,4,5,6-Tetrahydrophthalimido)-zimtsäurederivat der Formeln Ia und/oder Ib gemäß Anspruch 1 und inerte Zusatzstoffe.

5. Herbizide Mittel gemäß Anspruch 5, enthaltend ein 5-(N-3,4,5,6-Tetrahydrophthalimido)-zimtsäurederivat der Formeln Ia und/oder Ib und weitere wirksame Bestandteile.

6. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines 5-(N-3,4,5,6-Tetrahydrophthalimido)-zimtsäurederivates Ia und/oder Ib gemäß Anspruch 1 behandelt.

7. Verwendung der 5-(N-3,4,5,6-Tetrahydrophthalimido)-zimtsäurederivate der Formel Ia und Ib gemäß Anspruch 1 als Herbizide.

## Claims

### Claims for the following Contracting States : BE, CH, DE, FR, GB, IT, LI, NL

1. A 5-(N-3,4,5,6-Tetrahydrophthalimido)-cinnamic acid derivative of the formula Ia or Ib

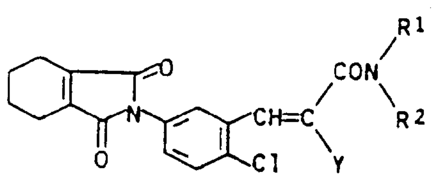

Ia

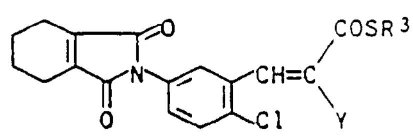

Ib

where

Y is chlorine, bromine or $C_1$-$C_4$-alkyl,

$R^1$ is hydrogen,

$C_1$-$C_8$-alkyl,

$C_2$-$C_4$-alkyl which carries a hydroxyl, $C_1$-$C_4$-alkoxy or $C_1$-$C_4$-alkylthio radical,

$C_3$-$C_6$-alkenyl,

$C_3$-$C_6$-alkynyl,

a phenyl ring or a benzyl radical, where the rings may carry from one to three of the following radicals: halogen, $C_1$-$C_4$-alkyl, $C_1$-$C_4$-alkoxy, $C_1$-$C_4$-haloalkyl, cyano, nitro, $C_1$-$C_4$-haloalkoxy, N-$C_1$-$C_4$-alkylamino, N, N-

di-$C_1$-$C_4$-alkylamino and/or $C_1$-$C_4$-alkylthio,

$R^2$ is $C_5$-$C_8$-alkyl,

$C_2$-$C_4$-alkyl which carries a hydroxyl, $C_1$-$C_4$-alkoxy or $C_1$-$C_4$-alkylthio radical,

$C_1$-$C_6$-alkoxy,

$C_3$-$C_6$-alkenyl,

$C_3$-$C_6$-alkenyloxy,

$C_3$-$C_6$-alkynyl,

$C_3$-$C_6$-alkynyloxy,

a phenyl, phenoxy, benzyl or benzyloxy radical, where these radicals may carry from one to three of the following substituents: halogen, $C_1$-$C_4$-alkyl, $C_1$-$C_4$-alkoxy, $C_1$-$C_4$-alkylthio, $C_1$-$C_4$-haloalkyl, cyano, nitro, $C_1$-$C_4$-haloalkoxy, N-$C_1$-$C_4$-alkylamino and/or N, N-di-$C_1$-$C_4$-alkylamino,

$R^3$ is $C_1$-$C_6$-alkyl or

$C_2$-$C_6$-alkyl which may carry from one to three of the following substituents: $C_1$-$C_4$-alkoxy, $C_1$-$C_4$-alkylthio and/or hydroxyl,

and $R^1$ is not hydrogen when Y is chlorine or bromine.

2. A process for the preparation of a compound Ia as claimed in claim 1, where Y is $C_1$-$C_4$-alkyl, wherein a nitrocinnamoyl chloride of the formula II

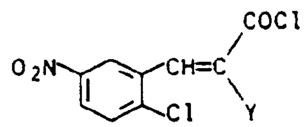

II

is reacted in a conventional manner in an inert organic solvent in the presence of a base with an amine of the formula III

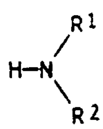

III

to give a nitrocinnamide IV

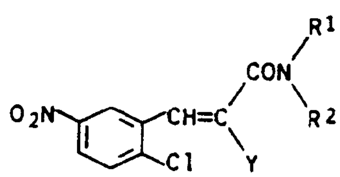

IV

the latter is then reduced to the amino compound V

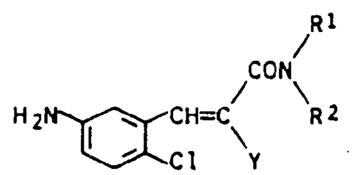

V

and V is condensed with 3,4,5,6-tetrahydrophthalic anhydride.

3. A process for the preparation of a compound Ia as claimed in claim 1, where Y is chlorine or bromine, wherein a nitrocinnamide of the formula IVa

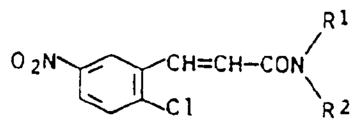

IVa

is halogenated in an inert organic solvent with chlorine or bromine to give a cinnamide of the formula IV

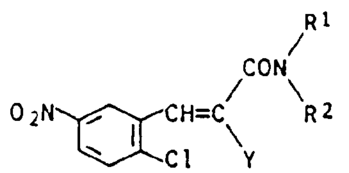

IV

the latter is then reduced (V) and condensed with 3,4,5,6-tetrahydrophthalic anhydride.

4. A process for the preparation of a compound Ia or Ib as claimed in claim 1, wherein a 5-(N-3,4,5,6-tetrahydrophthalimido)-cinnamoyl chloride of the formula VI

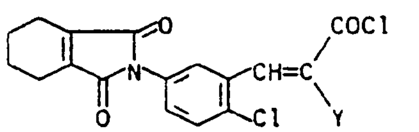

VI

is reacted in a conventional manner in an inert organic solvent either with a mercaptan of the formula VII

$$H\text{-}S\text{-}R^3 \qquad VII$$

or with an amine of the formula III.

5. A herbicide containing a 5-(N-3,4,5,6-tetrahydrophthalimido)-cinnamic acid derivative of the formulae Ia and/or Ib as claimed in claim 1 and inert additives.

6. A herbicide as claimed in claim 5, containing a 5-(N-3,4,5,6-tetrahydrophthalimido)-cinnamic acid derivative of the formulae Ia and/or Ib and further active components.

7. A method for controlling undesirable plant growth, wherein the undesirable plants and/or their habitat are treated with a herbicidal amount of a 5-(N-3,4,5,6-tetrahydrophthalimido)-cinnamic acid derivative Ia and/or Ib as claimed in claim 1.

8. Use of a 5-(N-3,4,5,6-tetrahydrophthalimido)-cinnamic acid derivative of the formulae Ia and/or Ib as claimed in claim 1 as a herbicide.

**Claims for the following Contracting State : ES**

1. A process for the prepration of a 5-(N-3,4,5,6-tetrahydrophthalimido)-cinnamic acid derivate of the formula Ia

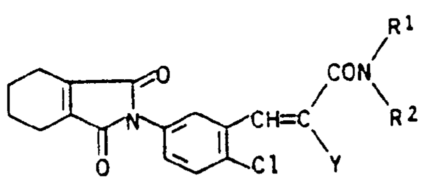

Ia

where

Y is $C_1$-$C_4$-alkyl;

$R^1$ is hydrogen,

$C_1$-$C_8$-alkyl,

$C_2$-$C_4$-alkyl which carries a hydroxyl, $C_1$-$C_4$-alkoxy or $C_1$-$C_4$-alkylthio radical,

$C_3$-$C_6$-alkenyl,

$C_3$-$C_6$-alkynyl,

a phenyl ring or a benzyl radical, where the rings may carry from one to three of the following radicals:

halogen, $C_1$-$C_4$-alkyl, $C_1$-$C_4$alkoxy, $C_1$-$C_4$-haloalkyl, cyano, nitro, $C_1$-$C_4$-haloalkoxy, N-$C_1$-$C_4$-alkylamino, N, N-di-$C_1$-$C_4$-alkylamino and/or $C_1$-$C_4$-alkylthio,

$R^2$ is $C_5$-$C_8$-alkyl,

$C_2$-$C_4$-alkyl which carries a hydroxyl, $C_1$-$C_4$-alkoxy or $C_1$-$C_4$-alkylthio radical,

$C_1$-$C_6$-alkoxy,

$C_3$-$C_6$-alkenyl,

$C_3$-$C_6$-alkenyloxy,

$C_3$-$C_6$-alkynyl,

$C_3$-$C_6$-alkynyloxy,

a phenyl, phenoxy, benzyl or benzyloxy radical, where these radicals may carry from one to three of the following substituents: halogen, $C_1$-$C_4$-alkyl, $C_1$-$C_4$-alkoxy, $C_1$-$C_4$-alkylthio, $C_1$-$C_4$-haloalkyl, cyano, nitro, $C_1$-$C_4$-haloalkoxy, N-$C_1$-$C_4$-alkylamino and/or N, N-di-$C_1$-$C_4$-alkylamino,

wherein a nitrocinnamoyl chloride of the formula II

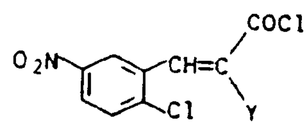 II

is reacted in a conventional manner in an inert organic solvent in the presence of a base with an amine of the formula III

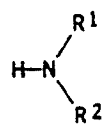 III

to give a nitrocinnamide IV

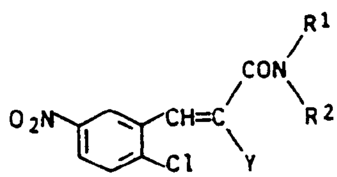 IV

the latter is then reduced to an amino compound V

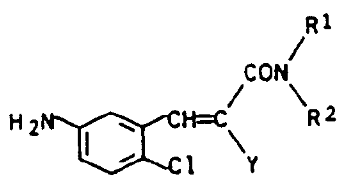 V

and V is condensed with 3,4,5,6-tetrahydrophthalic anhydride.

2. A process for the preparation of a compound Ia as claimed in claim 1, where Y is chlorine or bromine and $R^1$ is not hydrogen, wherein a nitrocinnamide of the formula IVa

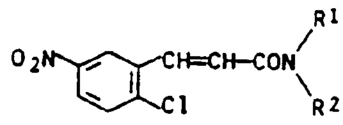 IVa

is halogenated in an inert organic solvent with chlorine or bromine to give a cinnamide of the formula IV

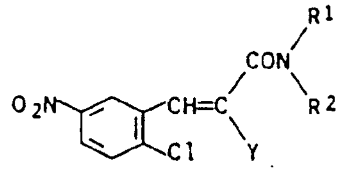

IV

the latter is then reduced (V) and condensed with 3,4,5,6-tetrahydrophthalic anhydride.

3. A process for the preparation of a compound Ia as claimed in claims 1 and 2 or Ib

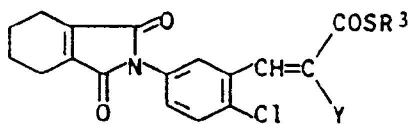

Ib

where

Y is chlorine, bromine or $C_1$-$C_4$-alkyl,

$R^3$ is $C_1$-$C_6$-alkyl or

$C_2$-$C_6$-alkyl which may carry from one to three of the following substituents: $C_1$-$C_4$-alkoxy, $C_1$-$C_4$-alkylthio and/or hydroxyl,

wherein a 5-(N-3,4,5,6-tetrahydrophthalimido)-cinnamoyl chloride of the formula VI

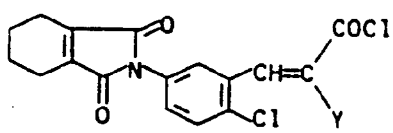

VI

is reacted in a conventional manner in an inert organic solvent either with a mercaptan of the formula VII

$H$-$S$-$R^3$ VII

or with an amine of the formula III.

4. A herbicide containing a 5-(N-3,4,5,6-tetrahydrophthalimido)-cinnamic acid derivative of the formulae Ia and/or Ib as claimed in claim 1 and inert additives.

5. A herbicide as claimed in claim 5, containing a 5-(N-3,4,5,6-tetrahydrophthalimido)-cinnamic acid derivative of the formulae Ia and/or Ib and further active components.

6. A method for controlling undesirable plant growth, wherein the undesirable plants and/or their habitat are treated with a herbicidal amount of a 5-(N-3,4,5,6-tetrahydrophthalimido)-cinnamic acid derivative Ia and/or Ib as claimed in claim 1.

7. Use of a 5-(N-3,4,5,6-tetrahydrophthalimido)-cinnamic acid derivative of the formulae Ia and/or Ib as claimed in claim 1 as a herbicide.

## Revendications

### Revendications pour les Etats contractants suivants : BE, CH, DE, FR, GB, IT, LI, NL

1. Dérivés d'acide 5-(N-3,4,5,6-tétrahydrophtalimido)-cinnamique des formules Ia et Ib

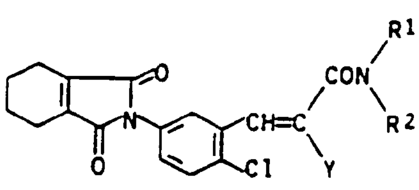

Ia

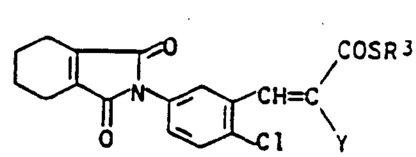

Ib

dans lesquelles les substituants ont les significations suivantes :

Y, chlore, brome ou un groupe alkyle en C 1-C 4

$R^1$, hydrogène,

un groupe alkyle en C 1-C 8,

un groupe alkyle en C 2-C 4 portant un reste hydroxy,

alcoxy en C 1-C 4 ou alkylthio en C 1-C 4,

un groupe alcényle en C 3-C 6,

un groupe alcynyle en C 3-C 6, un noyau phénylique ou un reste benzyle, les noyaux pouvant porter un à trois des restes suivants : halogène, alkyle en C 1-C 4, alcoxy en C 1-C 4, halogénalkyle en C 1-C 4, cyano, nitro, halogénalcoxy en C 1-C 4, N-alkylamino en C 1-C 4, N,N-di-alkylamino en C 1-C 4 et/ou alkylthio en C 1-C 4,

$R^2$, un groupe alkyle en C 5-C 8,

un groupe alkyle en C 2-C 4 qui porte un reste hydroxy, alcoxy en C 1-C 4 ou alkylthio en C 1-C 4,

un groupe alcoxy en C 1-C 6,

un groupe alcényle en C 3-C 6,

un groupe alcényloxy en C 3-C 6,

un groupe alcynyle en C 3-C 6,

un groupe alcylyloxy en C 3-C 6,

un reste phényle, phénoxy, benzyle ou benzyloxy, ces restes pouvant porter un à trois des substituants suivants : halogène, alkyle en C 1-C 4, alcoxy en C 1-C 4, alkylthio en C 1-C 4, halogènalkyle en C 1-C 4, cyano, nitro, halogènealcoxy en C 1-C 4, N-alkylamino en C 1-C 4 et/ou N,N-di-alkylamino en C 1-C 4,

$R^3$, un groupe alkyle en C 1-C 6,

un groupe alkyle en C 2-C 6, pouvant porter un à trois des substituants suivants : alcoxy en C 1-C 4, alkylthio en C 1-C 4 et/ou hydroxy,

$R^1$ ne représentant pas hydrogène lorsque Y a la signification de chlore et de brome.

2. Procédé de préparation des composés Ia selon la revendication 1, dans lesquels Y représente alkyle en C 1-C 4, caractérisé par le fait que l'on fait réagir, de manière en soi connue, dans un solvant organique inerte, en présence d'une base, un chlorure d'acide nitrocinnamique de formule II

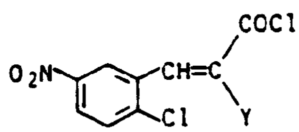

II

avec une amine de formule III

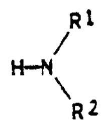

III

pour obtenir l'amide d'acide nitrocinnamique IV

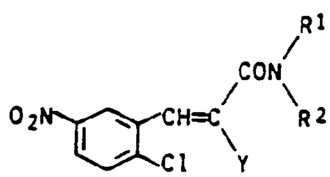

IV

on réduit ensuite en composé aminé V

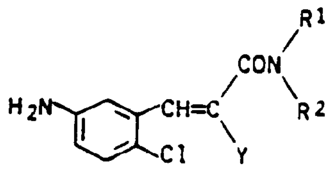

V

puis on condense V avec de l'anhydride 3,4,5,6-tétrahydrophtalique.

3. Procédé de préparation des composés Ia, selon la revendication 1, où Y représente chlore ou brome, caractérisé par le fait que l'on halogène un amide d'acide nitrocinnamique de formule IVa

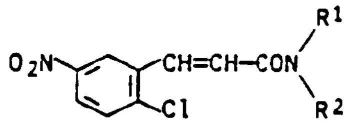

IVa

dans un solvant organique inerte, avec du chlore ou du brome, pour obtenir l'amide d'acide cinnamique de formule IV

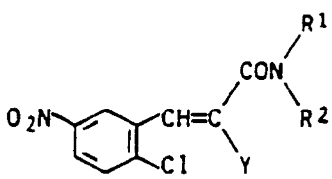

IV

on réduit ensuite (V) et on condense avec de l'anhdride 3,4,5,6-tétrahydrophtalique.

4. Procédé de préparation des composés Ia et Ib, selon la revendication 1, caractérisé par le fait que l'on fait réagir, de manière en soi connue, dans un solvant organique inerte, un chlorure d'acide 5-(N-3,4,5,6-tétrahydrophtalimido)-cinnamique de formule VI

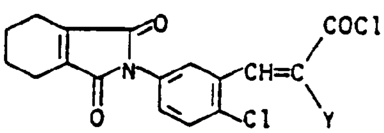

VI

soit avec un mercaptan de formule VII

$$H\text{-}S\text{-}R^3 \qquad VII$$

soit avec une amine de formule III.

5. Herbicide selon la revendication 5, contenant un dérivé d'acide 5-(N-3,4,5,6-tétrahydrophtalimido) cinnamique des formules Ia et/ou Ib et des additifs inertes.

6. Herbicide selon la revendication 5, contenant un dérivé d'acide 5-(N-3,4,5,6-tétrahydrophtalimido) cinnamique des formules Ia et/ou Ib et d'autres composants efficaces.

7. Procédé de lutte contre la croissance de plantes indésirables, caractérisé par le fait que l'on traite les plantes indésirables et/ou leur biotope avec une quantité efficace au point de vue herbicide d'un dérivé d'acide 5-(N-3,4,5,6-tétrahydrophtalimido)-cinnamique Ia et/ou Ib, selon la revendication 1.

8. Utilisation des dérivés d'acide 5-(N-3,4,5,6-tétrahydrophtalimido)-cinnamique des formules Ia et Ib, selon la revendication 1, commes herbicides.

**Revendications pour l'Etat contractant suivant : ES**

1. Procédé de préparation des dérivés d'acide 5-(N-3,4,5,6-tétrahydrophtalimido)- cinnamique de formule Ia

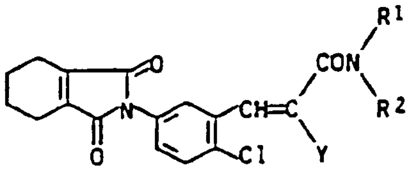

Ia

dans laquelle les substituants ont les significations suivantes :

Y, un groupe alkyle en C 1-C 4

$R^1$, hydrogène,

un groupe alkyle en C 1-C 8,

un groupe alkyle en C 2-C 4 portant un reste hydroxy,

alcoxy en C 1-C 4 ou alkylthio en C 1-C 4,

un groupe alcényle en C 3-C 6,

un groupe alcynyle en C 3-C 6, un noyau phénylique ou un reste benzyle, les noyaux pouvant porter un à trois des restes suivants : halogène, alkyle en C 1-C 4, alcoxy en C 1-C 4, halogénalkyle en C 1-C 4, cyano, nitro, halogénalcoxy en C 1-C 4, N-alkylamino en C 1-C 4, N,N-di-alkylamino en C 1-C 4 et/ou alkylthio en C 1-C 4,

$R^2$, un groupe alkyle en C 5-C 8,

un groupe alkyle en C 2-C 4 qui porte un reste hydroxy, alcoxy en C 1-C 4 ou alkylthio en C 1-C 4,

un groupe alcoxy en C 1-C 6,

un groupe alcényle en C 3-C 6,

un groupe alcényloxy en C 3-C 6,

un groupe alcynyle en C 3-C 6,

un groupe alcylyloxy en C 3-C 6,

un reste phényle, phénoxy, benzyle ou benzyloxy, ces restes pouvant porter un à trois des substituants suivants : halogène, alkyle en C 1-C 4, alcoxy en C 1-C 4, alkylthio en C 1-C 4, halogènalkyle en C 1-C 4, cyano, nitro, halogènealcoxy en C 1-C 4, N-alkylamino en C 1-C 4 et/ou N,N-di-alkylamino en C 1-C 4,

caractérisé par le fait que l'on fait réagir, de manière en soi connue, dans un solvant organique inerte, en présence d'une base, un chlorure d'acide nitrocinnamique de formule II

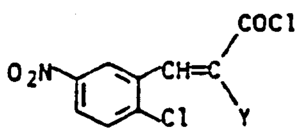

II

avec une amine de formule III

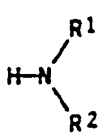

III

pour obtenir l'amide d'acide nitrocinnamique IV

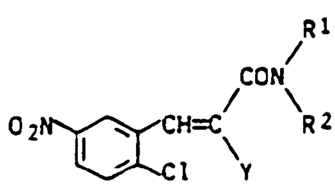

IV

on réduit ensuite en composé aminé V

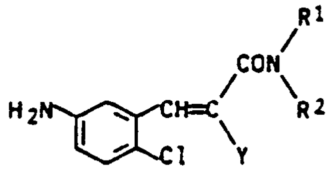

V

puis on condense V avec de l'anhydride 3,4,5,6-tétrahydrophtalique.

2. Procédé de préparation des composés Ia, selon la revendication 1, où Y représente chlore ou brome, et $R^1$ pas hydrogène caractérisé par le fait que l'on halogène un amide d'acide nitrocinnamique de formule IVa

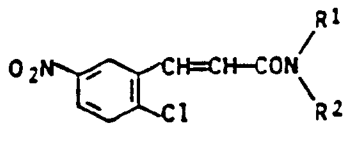

IVa

dans un solvant organique inerte, avec du chlore ou du brome, pour obtenir l'amide d'acide cinnamique de formule IV

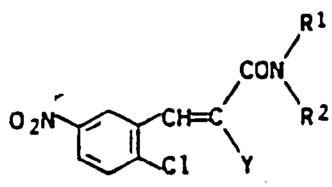

IV

on réduit ensuite (V) et on condense avec de l'anhdride 3,4,5,6-tétrahydrophtalique.

3. Procédé de préparation de composés Ia selon les revendications 1 et 2 et Ib

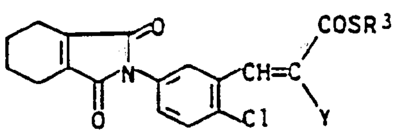

Ib

dans laquelle les substituants ont les significations suivantes :

Y, chlore, brome ou groupe alkyle en C 1-C 4

$R^3$, un groupe alkyle en C 1-C 6,

un groupe alkyle en C 2-C 6, pouvant porter un à trois des substituants suivants : alcoxy en C 1-C 4, alkythio en C 1-C 4 et/ou hydroxy.

caractérisé par le fait que l'on fait réagir, de manière en soi connue, dans un solvant organique inerte, un chlorure d'acide 5-(N-3,4,5,6-tétrahydrophtalimido)-cinnamique de formule VI

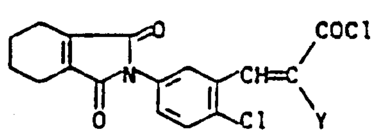

VI

soit avec un mercaptan de formule VII

$$H\text{-}S\text{-}R^3 \qquad VII$$

soit avec une amine de formule III.

4. Herbicide selon la revendication 1, contenant un dérivé d'acide 5-(N-3,4,5,6-tétrahydrophtalimido) cinnamique des formules Ia et/ou Ib et des additifs inertes.

5. Herbicide selon la revendication 5, contenant un dérivé d'acide 5-(N-3,4,5,6-tétrahydrophtalimido) cin-

namique des formules Ia et/ou Ib et d'autres composants efficaces.

6. Procédé de lutte contre la croissance de plantes indésirables, caractérisé par le fait que l'on traite les plantes indésirables et/ou leur biotope avec une quantité efficace au point de vue herbicide d'un dérivé d'acide 5-(N-3,4,5,6-tétrahydrophtalimido)-cinnamique Ia et/ou Ib, selon la revendication 1.

7. Utilisation des dérivés d'acide 5-(N-3,4,5,6-tétrahydrophtalimido)-cinnamique des formules Ia et Ib, selon la revendication 1, commes herbicides.